# EUROPÄISCHE PATENTSCHRIFT

(11) **EP 3 215 055 B1**
(45) Veröffentlichungstag und Bekanntmachung des Hinweises auf die Patenterteilung: **05.01.2022**
(21) Anmeldenummer: 15790534.0
(22) Anmeldetag: 03.11.2015
(51) Int. Cl.: A61F 2/07, A61F 2/852, A61F 2/90, A61F 2/89, A61F 2/06

(54) **MODULARES STENTGRAFT-SYSTEM**
MODULAR STENT GRAFT SYSTEM
SYSTÈME D'ENDOPROTHÈSE COUVERTE MODULAIRE

(30) Priorität: 04.11.2014 DE 102014116012
(43) Veröffentlichungstag der Anmeldung: 13.09.2017
(73) Patentinhaber: JOTEC GmbH, 72379 Hechingen (DE)
(72) Erfinder: BARTHOLD, Franz-Peter, 72336 Balingen (DE); BOGENSCHUETZ, Thomas, 72379 Hechingen-Stein (DE)
(74) Vertreter: Witte, Weller & Partner Patentanwälte mbB
(86) Internationale Anmeldenummer: PCT/EP2015/075631
(87) Internationale Veröffentlichungsnummer: WO 2016/071357

(56) Entgegenhaltungen:
- EP-A1- 1 759 666
- EP-A2- 1 177 780
- WO-A1-2008/130572
- DE-A1-102012 100 839
- US-A1- 2010 312 326

## Beschreibung

Die vorliegende Erfindung betrifft ein modulares Stentgraft-System zur Einführung in ein Blutgefäß eines zu behandelnden Patienten.

Derartige sind im Stand der Technik bspw. aus der DE 103 37 739.5 bekannt. DE 10 2012 100839 A1 offenbart eine Gefässprothese für einen Aortenbogen bestehend aus verschiedenen Stentgraftabschnitten, die fest miteinander verbunden sind.

WO 2008/130572 A1 offenbart ein modulares Stentgraft-System für den Einsatz in Stenosen.

Allgemein ist es bekannt, intraluminale Gefäßimplantate, die auch als endovaskuläre Gefäßstents/-stentgrafts bezeichnet werden, zur Behandlung von geschwächten, verletzten, gerissenen oder aneurysmatischen Gefäßen einzusetzen. Hierfür wird an der erkrankten oder verletzten Stelle des Gefäßes ein Gefäßimplantat bzw. Stentgraft freigesetzt, der die Funktionalität des ursprünglichen Gefäßes wieder herstellt bzw. die noch bestehende Gefäßintegrität unterstützt.

Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen, oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einer Ruptur der Arterie führen, mit der Folge, dass der Patient innerlich verblutet.

Die zur Behandlung derartiger Aneurysmen verwendeten selbstexpandierenden Gefäßimplantate bestehen im Allgemeinen aus einem hohlzylindrischen Metallrahmen bzw.-gerüst, dessen Mantelfläche mit einer Textil- oder Polymerfolie abgedeckt ist, so dass sich ein hohlzylindrischer Körper ergibt. Zur Implantation wird das Gefäßimplantat radial zusammengedrückt, so dass sich seine Querschnittsfläche deutlich verringert. Das Gefäßimplantat wird dann mit Hilfe eines Einführsystems in den Bereich des Aneurysma gebracht, wo es freigesetzt wird. Auf Grund der Federwirkung des Metallrahmens/-gerüsts expandiert das Gefäßimplantat wieder in seine ursprüngliche Form und spannt dabei seine Mantelfläche auf, die sich proximal und distal von dem Aneurysma innen in dem Blutgefäß verklemmt. Auf diese Weise fließt das Blut jetzt durch das Gefäßimplantat und eine weitere Belastung der Aussackung wird verhindert.

Der Metallrahmen solcher Gefäßimplantate besteht in der Regel bspw. aus einem Drahtgeflecht oder aus hintereinander angeordneten, mäanderförmig umlaufenden, sogenannten Stentfedern, die ggf. über Verbindungsstützen aus Draht miteinander verbunden sind, oder die lediglich über das Implantatmaterial miteinander verbunden sind. Das Drahtgeflecht bzw. die Stentfedern sind üblicherweise aus einem Shape-Memory-Material, in der Regel aus Nitinol, wodurch die Stentfedern nach Einbringung in ein Gefäß zur Freisetzung wieder in den expandierten Zustand übergehen und das Gefäßimplantat dadurch "aufspannen".

Aneurysmen treten häufig im Bereich der Baucharterie (Aorta abdominalis) oder Brustarterie (Aorta thoracica) auf. Zur Behandlung von Aneurysmen in der Bauch- oder Brustarterie ist es bereits bekannt, die Arterie durch Implantation eines Stents soweit zu stabilisieren, dass eine Ruptur des Gefäßes vermieden wird.

Aneurysmen können aber auch in dem sogenannten aufsteigenden Ast der Aorta (Aorta ascendens) auftreten. Der aufsteigende Ast der Aorta ist unmittelbar mit dem Herz verbunden. Ausgehend von der Aortenwurzel (Sinus aortae) verläuft der aufsteigende Ast in leicht gekrümmter Form vom Herz weg nach oben und geht dort in den Aortenbogen (Arcus aortae) über. Im Bereich des Aortenbogens zweigen die Kopfgefäße ab, u.a. die linke und die rechte Halsschlagader. Der Aortenbogen weist einen Kurvenverlauf von etwa 180° mit einem sehr engen Radius auf und verbindet den aufsteigenden Ast der Aorta mit der Brustarterie und im weiteren Verlauf mit der Baucharterie.

Nicht nur im Bereich des Aortenbogens ist es wichtig, vom Hauptgefäße abgehende Seiten-Gefäße durch die Positionierung des Gefäßimplantats nicht zu blockieren, weshalb viele Gefäßimplantate offene Zonen oder sogenannte Fenestrierungen aufweisen, über welche vom Gefäßimplantat abgehende Seitenäste, die in die Seiten-Gefäße hineinragen, eingeführt und am Gefäßimplantat fixiert werden können.

Ein Aneurysma oder eine Dissektion im aufsteigenden Ast der Aorta wird bis heute in einem invasiven offenen Eingriff behandelt. Eine solche Operation macht bisher regelmäßig zwei große, zeitlich getrennte Eingriffe erforderlich, und stellt einen sehr großen und aufwändigen und damit gefährlichen Eingriff dar, da nicht nur das Herz, sondern auch das Gehirn und die Bauchorgane des Patienten einer hypothermen Perfusion, d.h. also einer künstlichen, kalten extrakorporalen Durchblutung, bzw. einem hypothermen Durchblutungsstillstand unterzogen werden müssen. Mit einem solchen Eingriff sind jedoch nur wenige Herzchirurgen an erfahrenen Zentren vertraut.

Nach wie vor besteht daher ein großes Bedürfnis nach Stent-/Stentgraftsystemen, bzw Gefäßimplantaten mit Hilfe derer der oben geschilderte Eingriff vereinfacht und zeitlich verkürzt werden könnte.

Aufgabe der vorliegenden Erfindung ist es daher, ein System bereitzustellen, mit welchem Aneurysmen, insbesondere im Bereich der aufsteigenden Aorta, des Aortenbogens und der absteigendem Aorta, schnell und unkompliziert behandelt werden kann, bzw. das die oben geschilderten Eingriffe auch weniger erfahrenen Herzchirurgen ermöglicht.

Erfindungsgemäß wird diese und andere Aufgaben gelöst durch ein Stentgraft-System mit einem ersten hohlzylindrischen Stentgraftkörper und zumindest einem zweiten hohlzylindrischen Stentgraftkörper, wobei der erste und der zweite Stentgraftkörper voneinander baulich getrennt sind, und wobei jeweils der erste und der zweite Stentgraftkörper ein erstes Ende, ein zweites Ende sowie eine Längsachse aufweist, wobei jeweils der erste und der zweite Stentgraftkörper einen Stentgraft-Abschnitt aufweist, der aus einem Stentgraft-Stützgerüst und einem daran angebrachten Prothesenmaterial gebildet ist, wobei jeweils der erste und der zweite Stentgraftkörper ferner einen Stent-Abschnitt aufweist, der aus einem von Prothesenmaterial freien Stent-Stützgerüst gebildet ist, und der ein erstes und ein zweites Stent-Abschnitt-Ende aufweist, wobei der Stent-Abschnitt mit seinem ersten Stent-Abschnitt -Ende jeweils mit dem Stentgraft-Abschnitt fest verbunden ist, und wobei der Stent-Abschnitt des ersten Stentgraftkörpers und der Stent-Abschnitt des zweiten Stentgraftkörpers derart ausgebildet sind, dass sie über jeweils ihr zweites Stent-Abschnitt -Ende zumindest teilweise ineinander einführbar sind zur Ausbildung eines von Prothesen-material freien gemeinsamen Stent-Abschnitts.

Die der Erfindung zugrunde liegende Aufgabe wird dadurch vollkommen gelöst.

Mit dem neuen Stentgraft-System wird erreicht, dass ein von Prothesenmaterial-freier Abschnitt auf einfache Weise geschaffen werden kann, über welche die abgehenden Gefäße weiterhin mit Blut versorgt werden können, wobei gleichzeitig die verletzten betroffenen Gefäße durch den sogenannten gecoverten Stentgraft-Abschnitt, also den Abschnitt, der Prothesenmaterial aufweist, durch diese gestützt werden. Die Einführung und Platzierung dieses Systems kann durch dessen zweiteiligen Aufbau präzise und einfach gehandhabt werden.

Dabei wird zunächst das eine Modul des Stentgraft-Systems, also der erste hohlzylindrische Stentgraftkörper eingeführt und derart korrekt platziert, dass sich vom Hauptgefäß abgehende Gefäße im vom Prothesenmaterial freien Stent-Abschnitt befinden, sowie im Anschluss das zweite Modul derart in das zu behandelnde Blutgefäß und teilweise in das erste Modul eingeführt, dass die beiden von Prothesenmaterial freien Stent-Abschnitte zumindest teilweise überschneiden. Das zweite, als letztes eingeführte Modul, bzw. dessen von Prothesenmaterial freier Abschnitt, expandiert somit innerhalb des von Prothesenmaterial freien Abschnitts des ersten Moduls, und verankert dadurch das zweite Modul, also den zweiten hohlzylindrischen Stentgraftkörper teilweise innerhalb des ersten hohlzylindrischen Stentgraftkörpers. Die nicht innerhalb des von Prothesenmaterial freien Stent-Abschnitts des ersten Moduls verankerten Abschnitte des zweiten Moduls bzw. zweiten hohlzylindrischen Stentgraftkörpers expandieren im zu behandelnden Gefäß und legen sich an dessen Gefäßwände.

Durch das erfindungsgemäße Stentgraft-System, also durch die Kombination zweier einzelnen, baulich voneinander getrennten, und nacheinander einzuführenden Modulen, die jeweils einen kurzen, mit Prothesenmaterial bedeckten - sogenannten "gecoverten" - Stentgraft-Abschnitt aufweisen, sowie einen im expandierten und freigesetzten Zustand zwischen diesen gecoverten Stentgraft-Abschnitten liegenden, von Prothesenmaterial freien - sogenannten "nicht-gecoverten" - Stent-Abschnitt, wird es dadurch auch bzw. beispielsweise bei einer Implantation in den Aortenbogen möglich, die drei Abschnitte der Aorta, also die Aorta ascendens (aufsteigenden Hauptschlagader), des Aortenbogens und der descendierenden Aorta (absteigenden Hauptschlagader) simultan zu behandeln. Damit kann auf die Resektion des Aortenbogens mit all den damit verbundenen komplexen Perfusionsanforderungen für Gehirn und unteren Körper verzichtet werden, und der Eingriff nur mit Durchtrennung des obersten Abschnitts der aufsteigenden Aorta in einer kurzen, ca. 10 -20 minütigen selektiven Kopfperfusionsphase oder hypothermen Stillstandsphase erfolgen, um die neue Gefäßprothese einzuführen und freizusetzen. Die Einführung und Freisetzung kann dabei einfach unter Sicht des Auges bzw. eines Angioskops erfolgen.

Erfindungsgemäß wird erstmals eine intraluminale Gefäßprothese bereitgestellt, mit welcher die Möglichkeit geschaffen wird, Eingriffe insbesondere am Aortenbogen, bzw. in der aufsteigenden Aorta, dem Aortenbogen und der absteigenden Aorta, chirurgisch zu vereinfachen und zeitlich deutlich zu verkürzen. Vorteilhafterweise können damit nicht nur hoch spezialisierte Herzchirurgen die weiter oben geschilderten Eingriffe am Aortenbogen vornehmen. Ferner kann die erfindungsgemäße Gefäßprothese auch bei schwer erkrankten Patienten und bei älteren Patienten mit bspw. altersbedingt beschädigten Aortenwandschichten und mit Nicht-Durchblutung von lebenswichtigen Organsystemen wie Gehirn oder Bauchorganen eingesetzt werden, da diese mit einem zeitlich verkürzten und chirurgisch vereinfachten Verfahren eingesetzt werden können.

Vorteilhafterweise wird dabei der von Prothesenmaterial freie Stent-Abschnitt im expandierten Zustand im Bereich des Aortenbogens freigesetzt. Dadurch wird gewährleistet, dass der Blutfluss in die abgehenden Gefäße, wie den Truncuns brachiocephalicus, die linke Aorta carotis communis, und die linke Arteria subclavia, nicht beeinträchtigt wird. Das durch den Aortenbogen und die hierin zu verankernde Gefäßprothesen-/Stentgraft-System fließende Blut kann diese durch die im ungecoverten Stent-Abschnitt vorliegenden Öffnungen der Gefäßprothese verlassen; gleichzeitig ist sicher gestellt, dass das Stentgraft-System über die im freigesetzten Zustand beider Module dann links und rechts vom gemeinsamen gebildeten ungecoverten Stent-Abschnitt liegenden Stentgraft-Abschnitte sicher im Gefäß verankert ist; dies kann einerseits über radiale Expansionskräfte der Stentgraft-Abschnitte und das dadurch bewirkte Anliegen der Gefäßprothese in diesen Bereichen an die Gefäßwand erzielt werden. Ggf. bzw. sofern erwünscht, kann diese Fixierung auch durch ein Vernähen des Stentgraft-Abschnitts erreicht werden, welcher gemäß einer spezifischen Ausführungsform dann keine - bzw. nur optional - mäanderförmig umlaufenden Ringe und Stützen aufweist, mit der Aortenwand.

Nachstehend werden einige hierin verwendeten Begriffe, die zwar für den Fachmann an sich und unter Heranziehung der vorliegenden Offenbarung klar sind, näher definiert:

Vorliegend wird unter einer "Stentfeder" wie eingangs diskutiert, jedes einstückige, ringförmige Element verstanden, das sich aufgrund dessen Materials komprimieren lässt, und sich nach Entfernung des Kompressionsdrucks wieder federartig expandieren kann. Unter "mäanderförmig" wird vorliegen jeder schlingen- bzw. schleifenförmige Verlauf" der Stentfeder bzw. des Stentdrahtes verstanden, wobei jede Stentfeder einstückig, d.h. aus einem mäanderförmig umlaufenden Stentfederring gebildet ist.

Eine "mäanderförmig umlaufende einstückige Stentfeder" ist in diesem Zusammenhang vorliegend ein sich federartig expandierendes und komprimierbares ringförmiges Stentelement, das einen wellenartigen Umlauf besitzt, wobei sich Wellenberg und Wellental, die eine Phase bilden, abwechseln.

Vorteilhafterweise ist dabei ein Spitzbogen jeweils aus zwei Schenkeln und einem zwischen den Schenkeln liegenden Scheitelpunkt bzw. Tiefstpunkt gebildet.

"Zumindest ein Spitzbogen" bedeutet vorliegend, dass der Seitenkörper durch die Ausstellung eines einzigen Spitzbogens einer Stentfeder aufgespannt wird, oder aber durch zwei oder mehr Spitzbögen. In einer bevorzugten Ausführungsform sind zwei Spitzbögen einer Stentfeder ausgestellt. Grundsätzlich bildet die Mehrzahl der mäanderförmig umlaufenden Spitzbögen die Stentfeder des Grundkörpers, und der bzw. die ausgestellte(n) Spitzbogen/Spitzbögen bildet die Abzweigungsstelle für den Seitenkörper.

Grundsätzlich werden bei Stentgrafts oder endoluminalen Prothesen allgemein sowie vorliegend zur Bezeichnung der jeweiligen Enden die Begriffe "distal" und "proximal" verwendet, wobei der Begriff "distal" der Teil bzw. das Ende bezeichnet wird, der/das in Bezug auf den Blutstrom weiter stromabwärts liegt. Der Begriff "proximal" hingegen bezeichnet, wieder in Bezug auf den Blutstrom, einen Teil bzw. das Ende, der/das in Bezug auf den Blutstrom weiter stromaufwärts liegt. Anders ausgedrückt bedeutet der Begriff "distal" in Richtung des Blutstroms, und der Begriff "proximal" der Richtung des Blutstroms entgegengesetzt. Bei Kathetern hingegen, bzw. Einführsystemen bezeichnet der Begriff "distal" das Ende des Katheters bzw. Einführsystems, das in den Patienten eingeführt wird, bzw. das vom Anwender ausgesehen am Entferntesten liegt, und der Begriff "proximal" das Ende, das sich dem Anwender näher zugewandt ist.

Entsprechend sind vorliegend die "proximale" und die "distale" Öffnung des Gefäßimplantats die Öffnungen, durch die hindurch der Blutfluss durch den hohlzylindrischen Körper der Gefäßimplantat gewährleistet wird: Wenn das erfindungsgemäße Gefäßimplantat in einem Blutgefäß wie beispielsweise der Aorta implantiert ist, fließt also das vom Herzen kommende Blut durch die proximale Öffnung des Gefäßimplantats, und verlässt das Gefäßimplantat durch deren distale Öffnungen.

Das Stentgraft-System bzw. dessen beide hohlzylindrische Grundköper können dabei über ihre Gesamtlänge hinweg einen einheitlichen Durchmesser, oder aber unterschiedliche Durchmesser aufweisen.

Definitionsgemäß sind die Stentfedern nicht direkt miteinander verbunden, und weisen untereinander keine verbindenden Schenkel oder Streben oder ähnliche Verbindungselemente auf. Die Stentfedern sind lediglich über das Implantatmaterial, an welches die Stentfedern angebracht sind, miteinander verbunden, wodurch eine "indirekte Verbindung" zwischen den Stentfedern geschaffen wird.

Vorliegend wird unter "Stent" jede Vorrichtung oder eine Struktur bezeichnet, die einer Prothese eine Expansionskraft und/oder eine stützende Funktion verleiht.

Der Ausdruck "Stentgraft" soll vorliegend - wie auch im Stand der Technik - eine Prothese bedeuten, die einen bzw. mehrere Stent (oder Stentfedern) sowie ein damit verbundenes Implantat("graft")-Material aufweist, das ein Lumen durch zumindest einen Abschnitt der Prothese bildet.

Dabei ist gemäß einer Ausführungsform des Stentgraft-Systems bevorzugt, wenn das Stützgerüst des Stentgraft-Abschnitts des ersten und/oder zweiten Stentgraftkörpers aus in seiner Längsrichtung in einem Abstand hintereinander angeordneten, jeweils mäanderförmig umlaufenden Stentfedern gebildet ist, und ein an den Stentfedern befestigtes und diese verbindendes Prothesenmaterial aufweist.

Gemäß einer weiteren Ausführungsform ist das Stützgerüst des Stent-graft-Abschnitts und des Stent-Abschnitts des ersten und/oder zweiten Stentgraftkörpers einstückig ausgebildet ist, und das Prothesenmaterial im Stentgraft-Abschnitt hieran als Beschichtungsfolie aufgebracht ist.

In diesem Zusammenhang ist bevorzugt, wenn das Prothesenmaterial ein Material aufweist, das ausgewählt ist aus einem Textil oder einem Polymer.

Insbesondere ist bevorzugt, wenn das Prothesenmaterial ein Material aufweist oder aus diesem gebildet ist, das ausgewählt ist aus Polyester, Polyurethan, Polystyrol, Polytetrafluorethylen ultrahochmolekulargewichtiges Polyethylen (UHMPE), oder Mischungen davon.

Dabei kann das Prothesenmaterial des ersten und zweiten Stentgraft-körpers gleich oder unterschiedlich sein.

Gemäß einer Ausführungsform des erfindungsgemäßen Stentgraft-Systems ist bevorzugt, wenn das Stentgraft-System zur Implantation in eine Aorta ausgebildet ist, insbesondere im Bereich der aufsteigenden Hauptschlagader, des Aortenbogens und der absteigenden Hauptschlagader ausgebildet ist, wobei das Stentgraft-System zur Einbringung in die Aorta von einem komprimierten Zustand in einen expandierten Zustand überführbar ist, und wobei der erste und der zweite Stentgraftkörper zur Verankerung des Stentgraft-Systems in der Aorta ausgebildet sind.

In diesem Zusammenhang kann in weiteren Ausführungsformen vorgesehen und bevorzugt sein, wenn der von Prothesenmaterial freie gemeinsame Stent-Abschnitt im expandierten Zustand im Bereich des Aortenbogens freisetzbar ist.

Bei dem erfindungsgemäßen Stentgraft-System ist in diesem Zusammenhang vorgesehen, wenn der gecoverte Stentgraft-Abschnitt des ersten Stentgraftkörpers distal der Arteria subclavia zu liegen kommt, und der nicht-gecoverte Stent-Abschnitt des ersten Stentgraftkörpers im Aortenbogen. Der gecoverte Stentgraft-Abschnitt des zweiten Stentgraftkörpers wird im proximalen Aortenbogen platziert.

Anders ausgedrückt befindet sich daher der Stentgraft-Abschnitt des ersten Stentgraftkörpers am distalen Endbereich des Stentgraft-Systems und kann daher in der zusammengesetzten Form der beiden Stentgraftkörper auch als distaler Stentgraft-Abschnitt bezeichnet werden; der Stentgraft-Abschnitt des zweiten Stentgraftkörpers befindet sich am proximalen Endbereich der Gefäßprothese, der daher - wiederum im zusammengesetzten Zustand der beiden Stentgraftkörper - einen proximaler Stentgraft-Abschnitt bildet.

Im freigesetzten Zustand, in dem die beiden einzelnen Stentgraftkörper durch ein teilweises Überlappen der jeweiligen Stent-Abschnitte miteinander verankert sind, bilden die beiden gecoverten Stentgraft-Abschnitte also die äußeren Enden der zusammengesetzten Gefäßprothese, wobei dadurch der Stentgraft-Abschnitt des ersten Stentgraftkörpers am distalen Ende der dann zusammensetzten Gefäßprothese vorgesehen ist, und der Stentgraft-Abschnitt des zweiten Stentgraftkörpers am proximalen Ende.

Gemäß einer bevorzugten Ausführungsform ist vorgesehen, wenn der Stentgraft-Abschnitt und der Stent-Abschnitt des ersten und/oder zweiten Stentgraftkörpers aus einem selbst-expandierenden Material sind oder ein solches aufweisen. Hierbei ist insbesondere bevorzugt, wenn das Material Nitinol ist.

Gemäß einer weiteren bevorzugten Ausführungsform weist der Stent-graft-Abschnitt des ersten und/oder zweiten Stenftgraftkörpers zwischen zwei, drei, vier und fünf, vorzugsweise drei, hintereinander angeordnete Stentfedern aus mäanderförmig umlaufenden Stützen auf. Diese Ringe sind über das Prothesenmaterial miteinander verbunden. Gemäß einer bevorzugten Ausführungsform sind die Stützen bzw. Stentfedern selber untereinander nicht durch Stege o.ä. miteinander verbunden, bzw. stehen nicht in unmittelbaren Kontakt miteinander. Diese Ausführungsform hat den Vorteil, dass der Fachmann die Länge des Stentgraft-Abschnitts den jeweiligen Gefäßgegebenheiten anpassen kann.

Ferner ist gemäß einer weiteren Ausführungsform vorgesehen, wenn der von Prothesenmaterial freie Stent-Abschnitt des ersten und/oder zweiten Stentgraft-körpers ein geflochtenes oder gezwirbeltes Drahtgeflecht aufweist.

Dabei wird unter "Drahtgeflecht" jede Ausbildung eines Stents verstanden, bei der verschiedene Draht-Stränge derart miteinander verflochten, ineinander verschlungen oder anderweitig verknüpft sind, dass sich eine Struktur mit Zonen, Bereichen oder Punkten bildet, an denen die Stränge übereinander liegen, und mit Zonen oder Bereichen, die frei von den Drahtsträngen sind und daher Öffnungen oder Fenster oder Maschen bilden.

Gemäß einer alternativen Ausführungsform ist der von Prothesenmaterial freie Stent-Abschnitt des ersten/und oder zweiten Stentgraft-Körpers ein Laser-geschnittenes Rohr. Auch diese Ausführungsform weist Maschen oder Öffnungen auf, über die das in der Aorta bzw. im Aortenbogen geführte Blut die Aorta in die abgehenden Gefäße, insbesondere der Truncus brachiocephalicus, die linke Aorta carotis communis, und die linke Arteria subclavia, verlassen kann und damit eine Durchblutung dieser Gefäße gewährleistet.

Gemäß einer besonders bevorzugten Ausführungsform weist der Stent-graft-Abschnitt des ersten und/oder zweiten Stentgraftkörpers eine Länge von zwischen ca. 20 mm und ca.100 mm auf, sowie der jeweils nicht-gecoverte Stent-Abschnitt eine Länge von zwischen ca. 30 mm und ca. 100 mm. Die jeweiligen Stentgraft-Abschnitte und Stent-Abschnitte des ersten und zweiten Stentgraftkörpers können dabei unterschiedliche Längen aufweisen, die sich im Rahmen der angegebenen Bereiche befinden.

Die vorliegende Beschreibung betrifft ferner ein Verfahren zur Freisetzung des erfindungsgemäßen Stentgraft-Systems, wobei das Verfahren die folgenden Schritte aufweist:
- Einbringen des ersten Stentgraftkörpers in eine Aorta eines Patienten im komprimierten Zustand, derart, dass der Stentgraft-Abschnitt des ersten Stentgraftkörpers vollständig distal der Arteria subclavia positioniert wird;
- Überführen des ersten Stentgraftkörpers in den expandierten Zustand derart, dass der von Prothesenmaterial freie Stent-Abschnitt des ersten Stentgraftkörpers im Bereich der Abgänge des Truncus brachiocephalus, der Arteria carotis communis, und der Arteria subclavia sinistra im Aortenbogenfreigesetzt wird,
- Einbringen des zweiten Stentgraftkörpers in die Aorta eines Patienten im komprimierten Zustand, derart, dass der Stentgraft-Abschnitt des zweiten Stentgraftkörpers vollständig proximal des Abgangs des Truncus brachiocephalus positioniert wird, und der von Prothesenmaterial freie Stent-Abschnitt des zweiten Stentgraftkörpers zumindest teilweise in den von Prothesenmaterial freien Stent-Abschnitt des ersten Stentgraftköprers eingeführt wird; und
- Überführen des zweiten Stentgraftkörpers in den expandierten Zustand derart, dass der von Prothesenmaterial freie Stent-Abschnitt des zweiten Stentgraftkörpers zumindest teilweise im von Prothesenmaterial freien Stent-Abschnitt des ersten Stentgraftkörpers verankert wird.

Mit diesem Verfahren wird sicher gestellt, dass die erfindungsgemäße Stentgraft-System derart positioniert wird, dass der sich überschneidende nicht-gecoverte Stent-Abschnitt den Blutfluss in die abgehenden Gefäße Truncus brachiocephalus, Arteria carotis communis, und Arteria subclavia sinistra ermöglicht.

Hierbei wird darauf geachtet, dass der gecoverte Stentgraft-Abschnitt des ersten Stentgraftkörpers knapp distal des Arteria subclavia Abgangs endet. Der nicht gecoverte Stent-Abschnitt wird nun im Aortenbogen freigesetzt, wobei die Drahtmaschen bzw. die Öffnungen des Laser-geschnittenen Stent-Abschnitts so weiträumig sind, dass keine Gefährdung im Sinn einer Verlegung der Abgänge der Kopf-Hals Gefäße (Truncuns brachiocephalicus, linke Aorta carotis communis, und linke Arteria subclavia besteht. Im Anschluss daran wird der zweite Stentgraftkörper derart freigesetzt, dass dessen gecoverter Stentgraft-Abschnitt proximal des Abgangs des Truncus brachiocephalicus positioniert wird. Dieser expandiert oder kann mit dem proximalen Aortenbogen vernäht werden. Dieses Verfahren hat den Vorteil, dass dann, wenn zuvor bspw. bereits der aufsteigende Anteil der Aorta ascendens ersetzt worden war, diese blutungsstillende Naht simultan die Gefäßprothese mit fassen.

Die erfindungsgemäße Stentgaft-System und das nicht beanspruchte Verfahren zur Einbringung bzw. Freisetzung bieten daher den Vorteil, dass ein im Bereich der Aorta ascendens vorliegender Einriss oder ein Aneurysma durch Resektion und konventionellen prothetischen Einsatz - vermittelt durch den Stentgraft-Abschnitt des zweiten Stentgraftkörpers - wie bisher beseitigt werden kann, und simultan eventuell verbleibende Einrisse der Intima in der proximalen Aorta descendens oder im Aortenbogen sicher stabilisieren werden können, und zwar ohne Rupturgefahr im Langzeitverlauf. Dies kann insbesondere auch dadurch erreicht werden, dass die Gefäßprothese bspw. 10% bis 20% größer bzw. länger als jeweils notwendig ausgebildet ist (sog. "Oversizing").

Damit kann insbesondere im Falle einer Dissektionserkrankung oder komplexen aneurysmatischen Erkrankung der thorakalen Aorta der operative und zeitliche Aufwand gegenüber bisher im Stand der Technik angewandten Operationen und Systemen bei einem vergleichbaren Ergebnis gedrittelt, und damit das Risiko des Eingriffs signifikant gesenkt werden kann.

Ferner betrifft die Verwendung des erfindungsgemäßen Stentgraft-Systems die Behandlung von Dissektionserkrankungen oder aneurysmatischen Erkrankungen, insbesondere der thorakalen Aorta.

Weitere Vorteile ergeben sich aus den Figuren und der nachstehenden Beschreibung bevorzugter Ausführungsbeispiele.

Ausführungsbeispiele der Erfindung sind in der Zeichnung dargestellt und werden in der nachfolgenden Beschreibung näher erläutert. Es zeigen:
Fig. 1 eine schematische Darstellung einer Ausführungsform eines ersten Stentgraftkörpers des erfindungsgemäßen Stentgraft-Systems (nicht maßstabsgetreu), im nicht-eingeführten, aber expandierten Zustand;
Fig. 2 eine schematische Darstellung eines zweiten Stentgraftkörpers des erfindungsgemäßen Stentgraft-Systems (nicht maßstabsgetreu), ebenfalls im nicht-eingeführten, aber expandierten Zustand;
Fig. 3 eine schematische Darstellung des zusammengesetzten Stentgraft-Systems, bei welchem der Stent-Abschnitt des ersten Stenftgraftkörpers und er Stent-Abschnitt des zweiten Stentgraftkörpers überlappend und teilweise ineinander eingeführt sind.
Fig. 4 eine schematische Darstellung des wie in Fig. 3 zusammengesetzten Stentgraft-Systems, positioniert und freigesetzt im Aortenbogen.

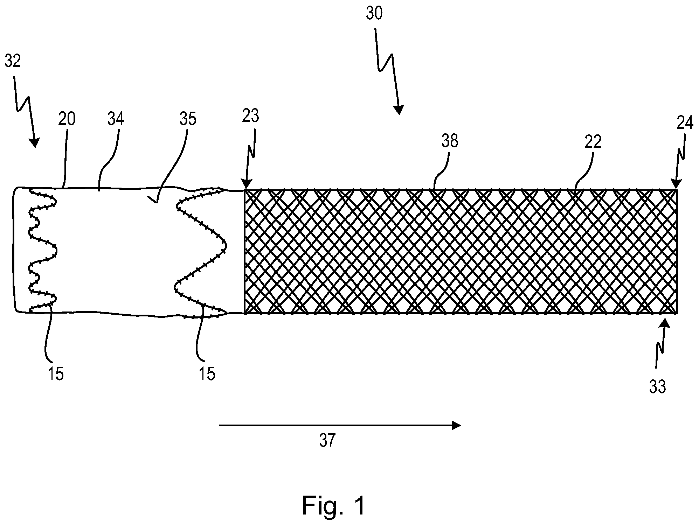

In Fig. 1 ist mit 30 insgesamt ein erster Stentgraftkörper 30 eines erfindungsgemäßen Stentgraft-Systems 10 gezeigt mit einem distalen Ende 32 und einem proximalen Ende 33, sowie einer Längsachse 37. Der erste Stentgraftkörper 30 weiet einen Stentgraft-Abschnitt 34 und einen Stent-Abschnitt 38 auf. Der Stentgraft-Abschnitt 34 wiederum weist ein Stentgraft-Stützgerüst 20 und ein daran angebrachtes Prothesenmaterial 35 auf. Das Stentgraft-Stützgerüst 20 umfasst in der in Fig. 1 gezeigten Ausführungsform hintereinander angeordnete Ringe bzw. Stentfedern 19 auf, die mäanderförmig um den Umfang des Stentgraftkörpers 30 umlaufen, und die über ein Prothesenmaterial 35 miteinander verbunden sind. Der Stent-Abschnitt 38 weist ebenfalls ein Stent-Stützgerüst 22 auf, ist frei von Prothesenmaterial und weist offene Zellen bzw. Öffnungen 40 auf. Mit den Bezugszeichen 23 und 24 sind jeweils ein erstes und ein zweites Stent-Abschnitt-Ende 23, 24 bezeichnet.

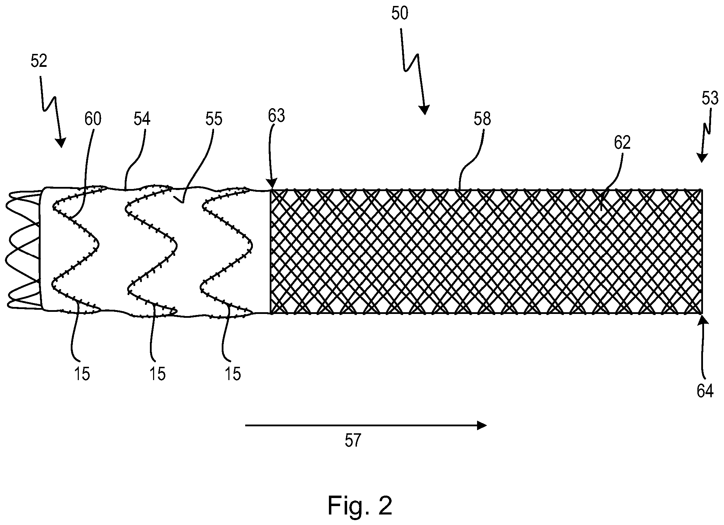

Die in Fig. 2 gezeigte Ausführungsform des zweiten Stentgraft-Körpers 50 besitzt ein erstes Stentgraftkörper-Ende 52 und ein zweites Stentgraftkörper-Ende 53 und eine Längsachse 57. Ferner hat der zweite Stentgraftkörper 50 einen Stentgraft-Abschnitt 54, der wiederum ein Stentgraft-Stützgerüst 60 aufweist, und der in dieser Ausführungsform wie der Stentgraft-Abschnitt 34 des ersten Stengraftkörpers 30 hintereinander angeordnete Ringe bzw. Stentfedern15 umfasst, die den Umfang des Stentgraftkörpers 50 mäanderförmig umlaufen. Der Stentgraft-Abschnitt 54 ist ferner mit einem die Ringe/Stentfedern 15 verbindenden Prothesenmaterial 55 bedeckt. Der zweite Stentgraftkörper 50 besitzt ferner einen Stent-Abschnitt 58, der aus einem von Prothesenmaterial freien Stent-Stützgerüst 62 gebildet ist, und der ein erstes Stent-Abschnitt-Ende 63 und ein zweites Stent-Abschnitt-Ende 64 aufweist.

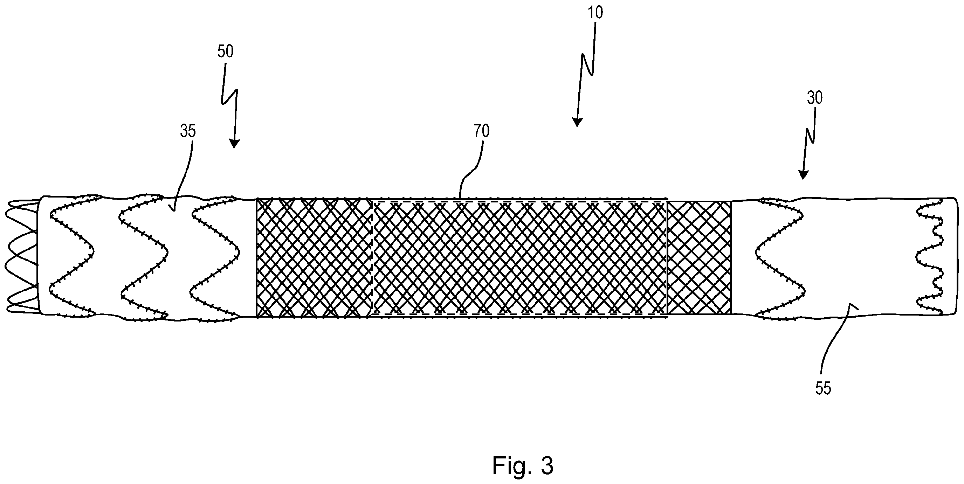

Fig. 3 zeigt nun die zusammengesetzte bzw. teilweise ineinander eingeführte Form des Stentgraft-Systems 10, wobei hier aus Gründen der Übersichtlichkeit nicht alle Bezugszeichen, die für die Figuren 1 und 2 vorliegend beschreibend verwendet werden, wiedergegeben sind.

Fig. 3 ist zu entnehmen, dass der erste Stentgraftkörper 30 über sein zweites Stentgraftköper-Ende 33, bzw. genauer über sein zweites Stent-Abschnitts-Ende 24, teilweise mit seinem Stent-Abschnitt 38 in den Stent-Abschnitt 58 des zweiten Stentgraftkörpers 50 über dessen zweites Stent-Abschnitts-Ende 64 eingeführt ist, so dass sich die beiden Stent-Abschnitte 38 und 58 teilweise überlappen, und so einen gemeinsamen Stent-Abschnitt 70 bilden. In der expandierten Form verankern sich die beiden Stent-Abschnitte 38, 58 durch die nach außen gerichtete Radialkraft, die von dem innen geführten Stent-Abschnitt 38 nach außen und somit in Richtung Stent-Abschnitt 58 ausgeübt wird, der sich wiederum gegen eine Gefäßwand drückt und das System 10 im zu behandelnden Gefäß fixiert.

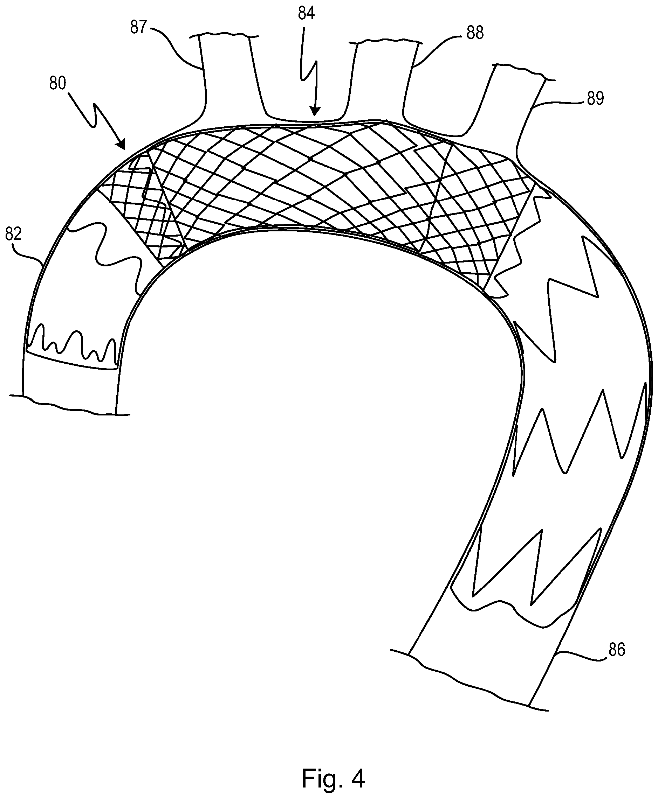

Fig. 4 zeigt schließlich die wie in Fig. 3 zusammengesetzte Ausführungsform des erfindungsgemäßen Stentgraft-Systems 10 in einem in eine Aorta 80 eingebrachten und dort expandierten Zustand: In Fig. 3 ist mit 82 ein Teil der aufsteigenden - ascendierenden - Aorta bezeichnet, mit 84 der Aortenbogen und mit 86 die absteigende - descendierende - Aorta. Wie aus Fig. 3 ersichtlich, gehen im Bereich des Aortenbogens 84 drei Gefäße 87, 88, und 89 ab, nämlich der Truncus brachiocephalus 87, die Arteria carotis communis 88, und die Arteria subclavia sinistra 89.

Fig. 4 ist ferner die Lage bzw. Positionierung der in Fig. 3 dargestellten zusammengesetzten Ausführungsform des erfindungsgemäßen Stentgraft-Systems 10 zu entnehmen: Zu erkennen ist, dass der Stentgraft-Abschnitt 34 des ersten Stentgraftkörpers 30 proximal des Truncus brachiocephalus positioniert ist, dass ferner der durch die beiden Stent-Abschnitte 38, 58, zusammengesetzte und von Prothesenmaterial freie gemeinsame Stent-Abschnitt 70 im Aortenbogen 84 freigesetzt ist und über seine Zellen bzw. Öffnungen oder Maschen 40 die abgehenden Gefäße 87, 88 und 89 mit Blut versorgen kann. Der Stentgraft-Abschnitt 54 des zweiten Stentgraftkörpers 50 ist distal der Arteria subclavia sinistra 89 positioniert.

Zur Einführung des erfindungsgemäßen Stentgraft-Systems 10 werden die Stentgraftkörper 30, 50 auf ein Einführsystem geladen (nicht gezeigt) und über eine entsprechende Hülle (nicht gezeigt) in einem komprimierten Zustand gehalten. Verfahren und Vorrichtungen zum Einführen von Gefäßprothesen sind dem Fachmann aus dem Stand der Technik geläufig. Zunächst wird der komprimiert gehaltene Stentgraftkörper 50 in die descendierende Aorta vorgeschoben, bis der komplette Stentgraft-Abschnitt 54 des zweiten Stentgraftkörpers 50 distal der Arteria subclavia liegt. Die korrekte Platzierung kann bspw. über entsprechende, an dem Stentgraftkörper 50 vorgesehene - bspw. röntgendichte - Marker kontrolliert werden. Nach korrekter Platzierung kann der Stentgraftkörper 50 nun durch Rückziehen der Hülle freigesetzt werden, wobei nach dem Stentgraft-Abschnitt 54 der von Prothesenmaterial freie - nicht-gecoverte - Stent-Abschnitt 58 im Aortenbogen 84 freigesetzt wird, wobei die Öffnungen bzw. Maschen 40 so weiträumig sind, dass keine Gefährdung im Sinn einer Verlegung der Abgänge der Kopf-Hals Gefäße 87, 88 und 89 (Truncuns brachiocephalicus, linke Aorta carotis communis, linke Arteria subclavia) besteht.

In einem nächsten Schritt wird der erste Stentgraftköprer 30 im komprimierten Zustand mit seinem Stent-Abschnitt 38 in den expandierten Stent-Abschnitt 58 des zweiten Stentgraftkörpers 50 zumindest teilweise vorgeschoben und der Stentgraftkörper 30 durch Rückziehen der komprimierenden Hülle, im Gefäß freigesetzt, derart, dass der Stent-Abschnitt 38 sich im Stent-Abschnitt 58 verankert, und der Stentgraft-Abschnitt 54 proximal des Abgangs des Truncus brachiocephalicus 87 zu liegen kommt.

Dem Fachmann wird klar sein, dass er die genauen Maße und räumlichen Anforderungen der einzelnen Stentgraftköper-Abschnitte, mithin also der Stentgraft-Abschnitt 34, 54, sowie der Stent-Abschnitte 38, 58 durch vorherige Untersuchung des zu behandelnden Patienten ermitteln und jeweils spezifisch umsetzen kann.

## Patentansprüche

1. Stentgraft-System (10) mit einem ersten hohlzylindrischen Stentgraftkörper (30) und zumindest einem zweiten hohlzylindrischen Stentgraftkörper (50), wobei der erste und der zweite Stentgraftkörper (30; 50) zwei baulich voneinander getrennte Stentgraftkörper sind, und wobei jeweils der erste (30) und der zweite (50) Stentgraftkörper ein erstes Ende (32; 52), ein zweites Ende (33; 53) sowie eine Längsachse (37; 57) aufweist, wobei jeweils der erste und der zweite Stentgraftkörper (30; 50) einen Stentgraft-Abschnitt (34; 54) aufweist, der aus einem Stentgraft-Stützgerüst (20; 60) und einem daran angebrachten Prothesenmaterial (35; 55) gebildet ist, wobei jeweils der erste und der zweite Stentgraftkörper (30; 50) ferner einen Stent-Abschnitt (38; 58) aufweist, der aus einem von Prothesenmaterial freien Stent-Stützgerüst (22; 62) gebildet ist, und der ein erstes (23; 63) und ein zweites (24; 64) Stent-Abschnitt-Ende aufweist, wobei der Stent-Abschnitt (38; 58) mit seinem ersten Stent-Abschnitt -Ende (23; 63) jeweils mit dem Stentgraft-Abschnitt (34; 54) fest verbunden ist, und wobei der Stent-Abschnitt (38) des ersten Stentgraftkörpers (30) und der Stent-Abschnitt (58) des zweiten Stentgraftkörpers (50) derart ausgebildet sind, dass sie über jeweils ihr zweites Stent-Abschnitt -Ende (24; 64) zumindest teilweise ineinander einführbar sind zur Ausbildung eines von Prothesenmaterial freien gemeinsamen Stent-Abschnitts (70).

2. Stentgraft-System (10) nach Anspruch 1, **dadurch gekennzeichnet, dass** das Stützgerüst (20; 60) des Stentgraft-Abschnitts (34; 54) des ersten und/oder zweiten Stentgraftkörpers (30; 50) aus in seiner Längsrichtung in einem Abstand hintereinander angeordneten, jeweils mäanderförmig umlaufenden Stentfedern (15) gebildet ist, und ein an den Stentfedern (15) befestigtes und diese verbindendes Prothesenmaterial (35; 55) aufweist.

3. Stentgraft-System (10) nach Anspruch 1, **dadurch gekennzeichnet, dass** das Stützgerüst (20; 60) des Stentgraft-Abschnitts (34; 54) und des Stent-Abschnitts (38; 58) des ersten und/oder zweiten Stentgraftkörpers (30; 50) einstückig ausgebildet ist, und das Prothesenmaterial (35; 55) im Stentgraft-Abschnitt (34; 54) hieran als Beschichtungsfolie aufgebracht ist.

4. Stentgraft-System (10) nach einem der Ansprüche 1 bis 3, **dadurch gekennzeichnet, dass** das Prothesenmaterial (35; 55) ein Material aufweist, das ausgewählt ist aus einem Textil oder einem Polymer.

5. Stentgraft-System (10) nach einem der Ansprüche 1 bis 4, **dadurch gekennzeichnet, dass** das Prothesenmaterial (35; 55) ein Material aufweist oder aus diesem gebildet ist, das ausgewählt ist aus Polyester, Polyurethan, Polystyrol, Polytetrafluorethylen ultrahochmolekulargewichtiges Polyethylen (UHMPE), oder Mischungen davon.

6. Stentgraft-System (10) nach einem der Ansprüche 1 bis 5, **dadurch gekennzeichnet, dass** es zur Implantation in eine Aorta (80), insbesondere im Bereich der aufsteigenden Hauptschlagader (82), des Aortenbogens (84) und der absteigenden Hauptschlagader (86) ausgebildet ist, wobei das Stentgraft-System (10) zur Einbringung in die Aorta (80) von einem komprimierten Zustand in einen expandierten Zustand überführbar ist, und wobei der erste (30) und der zweite (50) Stentgraftkörper zur Verankerung des Stentgraft-Systems (10) in der Aorta (80) ausgebildet sind.

7. Stentgraft-System (10) nach einem der Ansprüche 1 bis 6, **dadurch gekennzeichnet, dass** der Stentgraft-Abschnitt (34; 54) und der Stent-Abschnitt (38; 58) des ersten und/oder zweiten Stentgraftkörpers (30; 50) aus einem selbst-expandierenden Material sind oder ein solches aufweisen.

8. Stentgraft-System (30) nach einem der Ansprüche 1 bis 7, **dadurch gekennzeichnet, dass** der von Prothesenmaterial (35; 55) freie gemeinsame Stent-Abschnitt (38; 58) im expandierten Zustand im Bereich des Aortenbogens (84) freisetzbar ist.

9. Stentgraft-System (30) nach einem der Ansprüche 1 bis 8, **dadurch gekennzeichnet, dass** der Stentgraft-Abschnitt (34; 54) des ersten und/oder zweiten Stentgraftkörpers (30; 50) jeweils zwischen zwei und fünf mäanderförmig umlaufende Stentfedern (15) aufweist.

10. Stentgraft-System (10; 30) nach einem der Ansprüche 1 bis 9, **dadurch gekennzeichnet, dass** der von Prothesenmaterial (35; 55) freie Stent-Abschnitt (38; 58) des ersten und/oder zweiten Stentgraftkörpers (30; 50) ein geflochtenes oder gezwirbeltes Drahtgeflecht aufweist.

11. Stentgraft-System (10; 30) nach einem der Ansprüche 1 bis 9, **dadurch gekennzeichnet, dass** der von Prothesenmaterial (35; 55) freie Stent-Abschnitt (38; 58) des ersten und/oder zweiten Stentgraftkörpers (30; 50) ein Laser-geschnittenes Rohr ist.

## Claims

1. A stent-graft system (10) comprising a first hollow cylindrical stent-graft body (30) and at least one second hollow cylindrical stent-graft body (50), wherein the first and the second stent-graft bodies (30; 50) are two structurally separate stent-graft bodies, and wherein the first (30) and the second (50) stent-graft bodies each have a first end (32; 52), a second end (33; 53), and a longitudinal axis (37; 57), wherein the first and the second stent-graft bodies (30; 50) each comprise a stent-graft portion (34; 54) which is formed from a stent-graft scaffold (20; 60) and a prosthesis material (35; 55) applied thereon, wherein the first and the second stent-graft bodies (30; 50) each further comprise a stent portion (38; 58) which is formed from a stent scaffold (22; 62) which is free from prosthesis material and comprises a first (23; 63) and a second (24; 64) stent-portion end, wherein the stent portion (38; 58) is fixedly connected via its first stent-portion end (23; 63) to the stent-graft portion (34; 54) in each case, and wherein the stent portion (38) of the first stent-graft body (30) and the stent portion (58) of the second stent-graft body (50) are designed in such a way that they can be at least partially inserted into one another via their respective second stent-portion ends (24; 64) in order to form one shared stent portion (70) which is free from prosthesis material.

2. The stent-graft system (10) as claimed in claim 1, **characterized in that** the scaffold (20; 60) of the stent-graft portion (34; 54) of the first and/or the second stent-graft body (30; 50) is formed from stent springs (15) which are successively arranged and spaced apart from one another in the longitudinal direction of said scaffold, and each of which circumferentially meanders, and a prosthesis material (35; 55) which is fastened to the stent springs (15) and connects said stent springs.

3. The stent-graft system (10) as claimed in claim 1, **characterized in that** the scaffold (20; 60) of the stent-graft portion (34; 54) and of the stent portion (38; 58) of the first and/or second stent-graft body (30; 50) are formed as one piece, and the prosthesis material (35; 55) in the stent-graft portion (34; 54) is applied thereon as coating foil.

4. The stent-graft system (10) as claimed in one of claims 1 to 3, **characterized in that** the prosthesis material (35; 55) comprises a material which is selected from a textile or a polymer.

5. The stent-graft system (10) as claimed in one of claims 1 to 4, **characterized in that** the prosthesis material (35; 55) comprises a material or is formed from said material, which is selected from polyester, polyurethane, polystyrene, polytetrafluorethylene, ultra-high-molecular-weight polyethylene (UHMPE), or mixtures thereof.

6. The stent-graft system (10) as claimed in one of claims 1 to 5, **characterized in that** the stent-graft system is designed for implantation into an aorta (80), in particular in the region of the ascending aorta (82), the aortic arch (84), and the descending aorta (86), wherein the stent-graft system (10) can be transferred from a compressed state into an expanded state for insertion into the aorta (80), and wherein the first (30) and the second (50) stent-graft bodies are designed for anchoring the stent-graft system (10) in the aorta (80).

7. The stent-graft system (10) as claimed in one of claims 1 to 6, **characterized in that** the stent-graft portion (34; 54) and the stent portion (38; 58) of the first and/or second stent-graft body (30; 50) is made from a self-expanding material or comprises such a material.

8. The stent-graft system (30) as claimed in one of claims 1 to 7, **characterized in that** the shared stent portion (38; 58) which is free from prosthesis material (35; 55) can be released in the expanded state in the region of the aortic arch (84).

9. The stent-graft system (30) as claimed in one of claims 1 to 8, **characterized in that** the stent-graft portion (34; 54) of the first and/or second stent-graft body (30; 50) each comprises between two and five circumferentially meandering stent springs (15).

10. The stent-graft system (10; 30) as claimed in one of claims 1 to 9, **characterized in that** the stent portion (38; 58) of the first and/or second stent-graft body (30; 50) that is free from prosthesis material (35; 55) comprises a woven or twisted wire mesh.

11. The stent-graft system (10; 30) as claimed in one of claims 1 to 9, **characterized in that** the stent portion (38; 58) of the first and/or second stent-graft body (30; 50) that is free from prosthesis material (35; 55) is a laser-cut tube.

## Revendications

1. Système d'endoprothèse couverte (10) comprenant un premier corps d'endoprothèse couverte cylindrique creux (30) et au moins un deuxième corps d'endoprothèse couverte cylindrique creux (50), le premier et le deuxième corps d'endoprothèse couverte (30 ; 50) étant deux corps d'endoprothèse couverte structurellement séparés l'un de l'autre, et chacun des premier (30) et deuxième (50) corps d'endoprothèse couverte comportant une première extrémité (32 ; 52), une deuxième extrémité (33 ; 53) ainsi qu'un axe longitudinal (37 ; 57), chacun des premier et deuxième corps d'endoprothèse couverte (30 ; 50) comportant une portion d'endoprothèse couverte (34 ; 54) qui est formée à partir d'une structure de support d'endoprothèse couverte (20 ; 60) et d'un matériau de prothèse (35 ; 55) attaché à celle-ci, chacun des premier et deuxième corps d'endoprothèse couverte (30 ; 50) comportant en outre une portion d'endoprothèse (38 ; 58) qui est formée à partir d'une structure de support d'endoprothèse (22 ; 62) exempte de matériau de prothèse, et qui comporte une première (23 ; 63) et une deuxième (24 ; 64) extrémité de portion d'endoprothèse, la portion d'endoprothèse (38 ; 58) étant reliée de manière fixe par sa première extrémité de portion d'endoprothèse (23 ; 63) à la portion d'endoprothèse couverte (34; 54), et la portion d'endoprothèse (38) du premier corps d'endoprothèse couverte (30) et la portion d'endoprothèse (58) du deuxième corps d'endoprothèse couverte (50) étant conçues de manière à être insérées au moins partiellement l'une dans l'autre via leur deuxième extrémité de portion d'endoprothèse (24 ; 64) pour former une portion d'endoprothèse commune (70) exempte de matériau de prothèse.

2. Système d'endoprothèse couverte (10) selon la revendication 1, **caractérisé en ce que** la structure de support (20 ; 60) de la section d'endoprothèse couverte (34 ; 54) du premier et/ou du deuxième corps d'endoprothèse couverte (30 ; 50) est formée à partir de ressorts d'endoprothèse (15) disposés dans sa direction longitudinale à distance les uns derrière les autre et s'étendant chacun en forme de méandre, et comporte un matériau de prothèse (35 ; 55) fixé aux ressorts d'endoprothèse (15) et reliant ceux-ci.

3. Système d'endoprothèse couverte (10) selon la revendication 1, **caractérisé en ce que** la structure de support (20 ; 60) de la portion d'endoprothèse couverte (34 ; 54) et de la portion d'endoprothèse (38 ; 58) du premier et/ou du deuxième corps d'endoprothèse couverte (30 ; 50) est conçue d'une seule pièce et le matériau de prothèse (35 ; 55) dans la portion d'endoprothèse couverte (34 ; 54) est appliqué sur celui-ci en tant que film de revêtement.

4. Système d'endoprothèse couverte (10) selon l'une des revendications 1 à 3, **caractérisé en ce que** le matériau de prothèse (35 ; 55) comporte un matériau qui est choisi parmi un textile ou un polymère.

5. Système d'endoprothèse couverte (10) selon l'une des revendications 1 à 4, **caractérisé en ce que** le matériau de prothèse (35 ; 55) comporte un matériau, ou est formé de celui-ci, qui est choisi parmi le polyester, le polyuréthane, le polystyrène, le polytétrafluoroéthylène, l'ultra- polyéthylène à poids moléculaire élevé (UHMPE), ou des mélanges de ceux-ci.

6. Système d'endoprothèse couverte (10) selon l'une des revendications 1 à 5, **caractérisé en ce qu'**il est conçu pour être implanté dans une aorte (80), notamment au niveau de l'artère principale ascendante (82) de la crosse aortique (84) et de l'artère principale descendante (86), le système d'endoprothèse couverte (10) pouvant être passé d'un état comprimé à un état déployé en vue de l'introduction dans l'aorte (80), et le premier (30) et le deuxième (50) corps d'endoprothèse couverte étant conçus pour ancrer le système d'endoprothèse couverte (10) dans l'aorte (80).

7. Système d'endoprothèse couverte (10) selon l'une des revendications 1 à 6, **caractérisé en ce que** la portion d'endoprothèse couverte (34 ; 54) et la portion d'endoprothèse (38 ; 58) du premier et/ou du deuxième corps d'endoprothèse couverte (30 ; 50) sont en un matériau auto-expansible ou comporte un tel matériau.

8. Système d'endoprothèse couverte (30) selon l'une des revendications 1 à 7, **caractérisé en ce que** la portion d'endoprothèse commune (38 ; 58) exempte de matériau de prothèse (35 ; 55) peut être libérée à l'état déployé dans la zone de l'arc aortique (84).

9. Système d'endoprothèse couverte (30) selon l'une des revendications 1 à 8, **caractérisé en ce que** la portion d'endoprothèse couverte (34 ; 54) du premier et/ou du deuxième corps d'endoprothèse couverte (30 ; 50) comporte chacun entre deux et cinq ressorts d'endoprothèse (15) qui s'étendent circonférentiellement en forme de méandre.

10. Système d'endoprothèse couverte (10 ; 30) selon l'une des revendications 1 à 9, **caractérisé en ce que** la portion d'endoprothèse (38 ; 58), exempte de matériau de prothèse (35 ; 55), du premier et/ou du deuxième corps d'endoprothèse couverte (30 ; 50) comporte un treillis de fil métallique tressé ou torsadé.

11. Système d'endoprothèse couverte (10 ; 30) selon l'une des revendications 1 à 9, **caractérisé en ce que** la portion d'endoprothèse (38 ; 58), exempte de matériau de prothèse (35 ; 55), du premier et/ou du deuxième corps d'endoprothèse couverte (30 ; 50) est un tube découpé au laser.
